# EUROPEAN PATENT SPECIFICATION

(11) **EP 1 737 527 B1**
(45) Date of publication and mention of the grant of the patent: **29.09.2010**
(21) Application number: 05731961.8
(22) Date of filing: 05.04.2005
(51) Int. Cl.: A61M 25/00

(54) **METHODS FOR MODIFYING A BALLOON OF A CATHETER ASSEMBLY**
VERFAHREN ZUR MODIFIZIERUNG EINES BALLONS EINER KATHETERANORDNUNG
PROCEDES DE MODIFICATION DU BALLONNET D'UN ENSEMBLE CATHETER

(30) Priority: 07.04.2004 US 820316
(43) Date of publication of application: 03.01.2007
(60) Divisional application: 10007069.7
(73) Proprietor: Abbott Cardiovascular Systems Inc., Santa Clara, CA 95054 (US)
(72) Inventor: HOSSAINY, Syed, F.A., Fremont, CA 94555 (US); SRIDHARAN, Srinivasan, Bel Air, MD 21015 (US)
(74) Representative: McLeish, Nicholas Alistair Maxwell
(86) International application number: PCT/US2005/011488
(87) International publication number: WO 2005/099804

(56) References cited:
- WO-A-96/14895
- US-A- 5 893 840
- US-A- 6 010 480
- US-B1- 6 544 223
- US-B1- 6 585 926

## Description

### BACKGROUND

### Field of the Invention

This invention is directed to methods for modifying a balloon of a catheter assembly.

### Description of the State of the Art

Balloon catheters are used for a variety of different procedures, such as percutaneous transluminal coronary angioplasty (PTCA) and stent delivery. In PTCA, a catheter assembly having a balloon, integrated at an end segment of the catheter, is introduced percutaneously into the cardiovascular system of a patient via the brachial or femoral artery. The catheter is advanced through the coronary vasculature until the balloon portion is positioned across the occlusive lesion. Once in position across the lesion, the balloon is inflated to a predetermined size to radially compress against the atherosclerotic plague of the lesion to remodel the lumen wall. The balloon is then deflated to a smaller profile to allow the catheter to be withdrawn from the patients' vasculature.

In addition to remodeling of the vessel wall, balloons have been used to deliver a therapeutic substance at the occlusion site. Balloons having a porous wall membrane can be inflated with a fluid carrier including a therapeutic substance. Upon inflation of the balloon, the therapeutic fluid is expelled out from the porous wall membrane. Alternatively, a balloon can be coated with a therapeutic substance for delivery of the substance at the treatment site. One of the problems associated with porous balloon membrane is trauma that may be inflicted on the vessel walls caused by the ejection of the fluid out from the porous balloon membrane. If the fluid carrier is expelled at too high of a velocity, it can cause damage to the vessel wall, despite its medicinal properties. This has been referred to as the "jetting effect." To counterbalance the "jetting effect," the pores have been reduced in size to muffle the velocity of the therapeutic fluid. Minimizing the pore size has provided manufacturing challenges. Simple coating of balloons with a therapeutic substance has provided an inadequate platform for the local delivery of a drug to the occluded site. By the time the balloon reaches the intended site, most, if not all, of the drug will have washed away off of the balloon. Accordingly, there is a need to provide for an effective means of delivering a drug from a balloon.

Nonetheless, there is described in US 5,893,840 a balloon catheter which includes body affecting chemicals within microcapsules on the exterior of the balloon either alone or with a stent. The coating releases from the balloon when the balloon is inflated into contact with the lumen to be treated. The device provides placement of the dosage required at the location in need of treatment. A sheath may be employed over the balloon and microcapsules to prevent the microcapsules from being rubbed off during delivery through a body lumen.

US 6,010,480 describes an expansible balloon catheter having at least a first exterior surface with a given coefficient of friction and a second exterior surface with a greater coefficient of friction. In a compact form only the first exterior surface is exposed to produce one coefficient of friction during transfer of the collapsed or uninflated balloon to and across a lesion. When inflated, the second surface dominates the first surface and produces a second coefficient of friction.

WO 96/14895 describes an inflatable balloon for a catheter including a coating which causes the balloon to prefer a predetermined, low profile configuration, such as a trifold configuration, when deflated. The balloon has a wall which has an exterior polymeric coating. The coating is set while the balloon is in the predetermined low profile deflated configuration so that after inflation, the coating acts to urge the balloon to return to the low profile configuration as the balloon is deflated.

US 6,544,223 describes a drug delivery device for delivering therapeutic agents and method of making such a device. The device including an inflatable balloon having a plurality of holes formed in the wall of the balloon. A microporous coating covers a portion of the outer surface of the wall of the balloon. The thickness of the coating and the size of the micropores can permit delivery of a substance from the micropores of the coating and into the tissue of a patient's lumen.

For stent delivery, a stent can be securely crimped on the balloon. The balloon can be the same balloon used for the remodeling of the vessel wall or a second stent delivery balloon can be introduced into the patient. At the designated site, the stent is deployed by the balloon, and then the balloon is deflated and withdrawn from the bore of the stent, leaving the stent to maintain vascular patentcy and optionally to deliver a therapeutic substance. A stent can be modified to deliver a therapeutic substance by a polymeric coating. Briefly, a polymer dissolved in a solvent and a therapeutic agent added thereto can be applied to the surface of a stent. The solvent is evaporated, leaving a polymeric coating, impregnated with a therapeutic substance, on the stent surface. A polymeric coating can increase the coefficient of friction between the stent and the balloon of a catheter assembly on which the stent is crimped for delivery. Additionally, some polymers have a "sticky" or "tacky" consistency. If the polymeric material either increases the coefficient of friction or adherers to the catheter balloon, the effective release of the stent from the balloon after deflation can be compromised. If the stent coating adheres to the balloon, the coating, or parts thereof, can be pulled off the stent during the process of deflation and withdrawal of the balloon following the placement of the stent. Adhesive, polymeric stent coatings can also experience extensive balloon sheer damage post-deployment, which could result in a thrombogenic stent surface and embolic debris. The stent coating can stretch when the balloon is expanded and may delaminate as a result of such shear stress. Accordingly, there is a need to eliminate or minimize damage caused to a coating of a stent by the delivery balloon. The embodiments of the present invention provide for methods to modify the balloon to achieve this as well as other results.

### BRIEF DESCRIPTION OF THE DRAWINGS

FIG. 1 illustrates a balloon integrated on a catheter assembly; the balloon is illustrated in a collapsed configuration, an under inflated state, an intended inflated state, and a hyper or over inflated state.
FIGs. 2 and 3 are SEM microphotographs showing ePTFE balloons after the immersion of the balloons in a solution of EVEROLIMUS.
FIG. 4 is an optical microphotograph comparison of dyed and non-dyed ePTFE balloons.

### SUMMARY

In accordance with the present invention, there is provided a method of modifying a balloon of a catheter assembly as recited in appended Claim 1, In some embodiments, the inflated state is greater than a range of an intended expanded configuration of the balloon. In other embodiments, the inflated state is less than a range of an intended expanded configuration of the balloon. The substance can be in a fluid form or carried by a fluid carrier, such as a solvent. If the substance is applied in a wet format, a drying step can accompany the step of applying the wet substance. The balloon can be reduced to a deflated state or to the collapsed configuration during the removal of the fluid.

### DETAILED DESCRIPTION

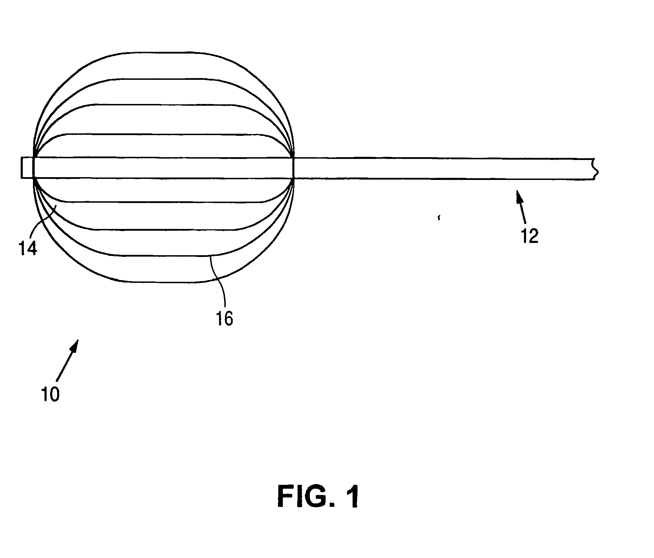

FIG. 1 illustrates a balloon 10 incorporated at an end segment of a catheter 12. The balloon 10 is intended to include any type enclosed member such as an elastic type member that is selectively inflatable to dilate from a collapsed configuration to a desired and controlled expanded configuration. The balloon 10 should also be capable of being deflated to a reduced profile or back to its original collapsed configuration. The balloon 10 can be made of any suitable type of material and can be of any thickness so long as the ability to modify the balloon and optimum performance capabilities of the balloon are not adversely compromised. Modification of the balloon will be discussed in detail below. Performance properties include high burst strength, good flexibility, high resistance to fatigue, an ability to fold, and ability to cross and re-cross a desired region of treatment or an occluded region in a body lumen, and a low susceptibility to defects caused by handling, among other possibilities. Porous is intended to include cavities or surface depots created by a manufacturing process (e. g., laser drilling or etching). Examples of materials that can be used include poly(tetrafluoroethylene)(PTFE), expanded poly(tetrafluoroethylene) (ePTFE), or expanded poly(ethylene). One variety of expanded poly(ethylene) that can be used includes expanded ultra-high molecular weight polyethylene, having molecular weight between about 500,000 and about 10,000,000 Daltons, Examples of some other porous materials that can be used to make a balloon include expanded poly(trifluoro ethylene) (e. g., EASYSTREET balloon available form Guidant Corp.), poly(urethanes), poly(amides), poly(esters), poly(ethylenes), including an ultra high molecular weight (polyethylene). Examples of poly(urethanes) include poly(ester urethanes), poly(ether urethanes), poly(silicone urethanes), and poly(carbonate urethanes). In particular, poly(urethane) products such as PELLETHANE or TECOTHANE can be used. Examples of some poly(esters) that can be used include poly(ethylene terephthalate) and poly(butylene terephthalate). Examples of some poly(amides) that can be used include NYLON and PEBAX. PELLETHANE is a trade name of a family of thermoplastic polyurethane elastomers having ether, ester, or caprolactone fragments. PELLETHAN products are available from Dow Chemical Co. of Midland, Michigan. TECOTHANE is a trade name of a family of thermoplastic aromatic poly(ether urethanes). TECOTHANE products are available from Thermedics Polymer Products Co. of Wilmington, Massachusetts. NYLON is a trade name of a family of poly(amides). NYLON products are available from E.I. DuPont deNemours Co. of Wilmington, Delaware. PEBAX is a trade name of a family of poly(ether)-block-poly(amide) copolymers. PEBAX products are available from Atofina Chemicals, Inc. of Philadelphia, Pennsylvania.

A non-porous material is used to make a balloon in which pores can be drilled using laser drilling or other forms of mechanical or chemical drilling. The drilling should not puncture the balloon wall but only leave depots or cavities on the surface of the balloon. The depth of drilling depends in part on the material from which the balloon is made and the thickness of the balloon wall.

FIG. 1 illustrates the balloon 10 in its collapsed configuration 14 as well as its intended deployment or expanded configuration 16. Collapsed configuration 14 is the state of complete deflation such as when no gas or fluid is introduced into the balloon 10. A balloon is inserted into a patient and maneuvered to the designated area of treatment in its collapsed configuration. Intended expanded configuration is defined as inflation of a balloon to a diameter or size within the range of its intended use or design. The intended expanded configuration is provided by the manufacturer of the balloon (or can be determined by one having ordinary skill in the art) and is intended to include the range of diameter of use or the range of pressure to be applied for the planned performance of the balloon. Under inflation is defined as any diameter between the collapsed configuration and the intended expanded configuration. Over or hyperinflation is defined as any diameter above intended expanded configuration but less than a diameter or size in which the balloon will be damaged or no longer suitable for its intended use. The term "inflation," "inflated," "inflated state," or "expanded" is to include, unless otherwise specified, under inflation, intended expanded configuration as well as hyperinflation.

The balloon can be modified with one or a combination of a drug or therapeutic substance, a polymer, or a blocking agent. Modification is intended to include deposition of the substance within the balloon wall membrane. In other words, the substance penetrates within the membrane from which the balloon is made. The substance, such as the blocking agent, can be in a dry powdered form, or can be a fluid form by itself or when mixed or dissolved in a solvent. Modification can be achieved by, for example, spraying or brushing a modifying substance on the balloon or, preferably, dipping the balloon in the solution of the substance. The substance can be dissolved, saturated or supersaturated in a solvent. The balloon is first inflated and then the modifying substance is applied to the balloon. For example, the balloon is first inflated and then immersed into a modifying substance or sprayed with the modifying substance. In some embodiments, the state of inflation should be maintained during the modification process. For example, if the balloon is hyper-inflated, during the course of the process, the balloon should remain hyper-inflated with no or only a negligible fluctuation in the balloon diameter or pressure applied in the balloon. In other embodiments, the state of inflation can be gradually increased or reduced during the modification process. For example, the state of inflation can be reduced from a hyper-inflated state to an under inflated state during the modification process.

In some embodiments, if a wet (e.g. solvent) application is employed, the state of inflation of the balloon should also be maintained during the drying process. That is, the state of inflation during the application of a solvent and a modifying agent is generally the same as the state of inflation during the evaporation of the solvent. In other embodiments, prior to or during the drying process, the state of inflation of the balloon can be modified to a different state. For example, the balloon can be modified at a hyper-inflated state and dried in its intended expanded configuration or an under inflated state; the balloon can be modified in its intended expanded configuration and can be dried in an under inflated state or in a hyper-inflated state; or the balloon can be modified in an under inflated state and dried in the state of intended expanded configuration or hyper-inflated configuration. In some embodiments, the drying can be conducted in a deflated state such that prior to or during the drying process, pressure applied to the balloon can be reduced negligibly or significantly so as to collapse the pores. In other embodiments, the drying process can be conducted a collapsed configuration. That is, subsequent to the modification of the balloon, fluid or air is removed from within the balloon and/or a vacuum pressure is applied so as to return the balloon back to its collapsed configuration. The balloon can then be dried. Drying or evaporation of the solvent can be expedited with the application of heat.

During the application of the modifying substance and/or during the drying process of a wet substance, in some embodiments, the balloon can be pulsed. Pulsing or pulsating is defined as increasing and/or decreasing the diameter or size of the balloon for one cycle or more. For example, the balloon can be pulsed in an under inflated state such that the pulsing action does not inflate the balloon to the intended expanded diameter state. Alternatively, the balloon can be pulsed from an under inflated state to the intended expanded state and back to the under inflated state during the coating procedure. This can be repeated more than once. In another example, the balloon can be pulsed from an intended expansion state to a hyper-inflated state. In another example, the balloon can be pulsed from the hyper-inflated state to the intended expanded configuration for more than one time. The pulsing action can carry into the drying stage or can be terminated prior to the drying stage. In some embodiments, the pulsing is done only in the drying stage and not the modification state.

In some embodiments, a gas, such as air or an inert gas (e.g., argon or nitrogen) can be applied to the balloon contemporaneously with the application of the modifying substance or subsequent to the termination of the applications of the substance. The temperature of the gas can depend on the volatility of the fluid or solvent carrier. In some embodiments, "volatile solvent" means a solvent that has a vapour pressure greater than 17.54 Torr (2.34 KPa) at ambient temperature, and "non-volatile solvent" means a solvent that has a vapour pressure less than or equal to 17.54 Torr (2.34 KPa) ambient temperature. A warm gas may be particularly suitable for embodiments in which the solvent employed in the composition is a non-volatile solvent (e.g., dimethylsulfoxide (DMSO), dimethylformamide (DMF), and dimethylacetamide (DMAC)). The temperature of the warm gas can be from about 25°C to about 200°C, more narrowly from about 40°C to about 90°C. In an embodiment of the present invention, a gas can be directed onto the balloon to inhibit evaporation of the solvent from the composition. Inhibition of evaporation of a solvent may be useful if the solvent is extremely volatile because the solvent may evaporate too quickly and not be capable of penetrating into the balloon membrane. In order to reduce the rate of evaporation of a solvent, a cool gas with a temperature of about less than 25°C can be used. The temperature of the gas can be, for example, significantly less than the boiling temperature of the solvent. The flow speed of the gas can be from about 300 feet/minute (91.5 meters/minute) to about 10,000 feet/minute (3047.85 meters/minute), more narrowly about 2500 feet/minute (761.96 meters/minute) to about 6000 feet/minute (1828.71 meters/minute). The gas can be applied for about 1 second to about 100 seconds, more narrowly for about 2 seconds to about 20 seconds. The application of the modifying substance and gas can be applied any number of cycles until the desired amount of substance is retained by the balloon. In some applications, the balloon can be rotated during the application of the substance and/or the drying stage.

Examples of drugs or therapeutic substances that can be used include any substance capable of having a therapeutic, prophylactic or diagnostic effect of a patient. Examples of therapeutic substances that can be used include antiproliferative substances such as actinomycin D, or derivatives and analogs thereof (manufactured by Sigma-Aldrich of Milwaukee, Wisconsin, or COSMEGEN available from Merck). Synonyms of actinomycin D include dactinomycin, actinomycin IV, actinomycin I₁, actinomycin X₁, and actinomycin C₁. The active agent can also fall under the genus of antineoplastic, anti-inflammatory, antiplatelet, anticoagulant, antifibrin, antithrombin, antimitotic, antibiotic, antiallergic and antioxidant substances. Examples of such antineoplastics and/or antimitotics include paclitaxel (e.g. TAXOL^{®} by Bristol-Myers Squibb Co., Stamford, Conn.), docetaxel (e.g. Taxotere^{®}, from Aventis S.A., Frankfurt, Germany) methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, and thrombin inhibitors such as ANGIOMAX (Biogen, Inc., Cambridge, Mass.). Examples of such cytostatic or antiproliferative agents include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ); calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents which may be appropriate include alpha-interferon, genetically engineered epithelial cells, tacrolimus, dexamethasone, and rapamycin and structural derivatives or functional analogs thereof, such as 40-O-(2-hydroxy)ethyl-rapamycin (known by the trade name of EVEROLIMUS available from Novartis), 40-O-(3-hydroxy)propyl-rapamycin, 40-O-[2-(2-hydroxy)ethoxy]ethyl-rapamycin, and 40-O-tetrazole-rapamycin.

A blocking agent is intended to reduce adhesion and/or friction between a polymer coated stent and the balloon so as to minimize balloon damage to the polymeric coating of a stent. In some embodiments, a blocking agent is intended to have a reverse effect, i.e., to increase adhesion and/or friction between a polymer coated stent or a bare stent and the balloon. This may be useful if the bare stent or the polymer used to make the coating is too slippery so as to not allow the stent to remain adequately crimped on the balloon. Examples of blocking agents that can be used include sucrose, poly(ethylene glycol)(PEG), poly(ethylene oxide)(PEO), solvent-soluble fluorinated polymers, block copolymers of bioabsorbable polymers with perfluorinated end chains, SILWET surfactants (available from Union Carbide Corp.), FLUORAD surfactants (available from 3M Co.), non-ionic surfactants having alkyl, perfluorinated, or silicone chains, fatty alcohols, waxes, fatty acid salts, mono-, di-, and triglycerides, cholesterol, lecithin, dextran, dextrin, esters and ethers of cellulose, e.g., carboxymethyl cellulose and cellulose acetate, cellulosics, maltose, glucose, mannose, trehalose, sugars, poly(vinyl alcohol)(PVA), poly(2-hydroxyethyl methacrylate), poly(N-vinyl-pyrrolidone)(PVP), silicone oil, paraffins, paraffin oil, and inorganic powders, such as talcum powder, calcium salt powder, and magnesium salt powder. Other carbohydrates such as starches and dextrose can also serve as a blocking agent. Hyaluronic acid can also be used to reduce friction and/or adhesion. In some embodiments, the blocking agent can simultaneously serve as a drug. Examples of such dual-function blocking agents include steroids, clobetasol, estradiol, dexamethasone, paclitaxel, rapamycin, (available from Wyeth Pharmaceuticals of Madison, New Jersey, under the name sirolimus), and structural derivative or functional analogs of rapamycin, such as 40-O-(2-hydroxy)ethyl-rapamycin (known by the trade name of EVEROLIMUS available from Novartis), 40-O-(3-hydroxy)propyl-rapamycin, 40-O-[2-(2-hydroxy)ethoxy]ethyl-rapamycin, and 40-O-tetrazole-rapamycin, and drugs with an octanol/water partition coefficient greater than 100.

Polymers that are hyrdrophilic or hydrophobic can be used to modify the balloon. These polymer can, in some embodiments, be combined with a drug and/or blocking agent. Examples of polymers that can be used include poly(ethylene-co-vinyl alcohol) (EVAL), poly(hydroxyvalerate), poly(L-lactic acid), polycaprolactone, poly(lactide-co-glycolide), poly(hydroxybutyrate), poly(hydroxybutyrate-co-valerate), polydioxanone, polyorthoester, polyanhydride, poly(glycolic acid), poly(D,L-lactic acid), poly(glycolic acid-co-trimethylene carbonate), polyphosphoester, polyphosphoester urethane; poly(amino acids), cyanoacrylates, poly(trimethylene carbonate), poly(iminocarbonate), co-poly(ether-esters) (e.g. PEO/PLA), polyalkylene oxalates, polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers, vinyl halide polymers and copolymers (such as polyvinyl chloride), polyvinyl ethers (such as polyvinyl methyl ether), polyvinylidene halides (such as polyvinylidene fluoride and polyvinylidene chloride), polyacrylonitrile, polyvinyl ketones, polyvinyl aromatics (such as polystyrene), polyvinyl esters (such as polyvinyl acetate), copolymers of vinyl monomers with each other and olefins (such as ethylene-methyl methacrylate copolymers, acrylonitrile-styrene copolymers, ABS resins, and ethylene-vinyl acetate copolymers), polyamides (such as Nylon 66 and polycaprolactam), alkyd resins, polycarbonates, polyoxymethylenes, polyimides, polyethers, epoxy resins, polyurethanes, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose.

In some embodiments, the balloon can be modified with a low adhesion polymer to prevent damage to a polymer coated stent. Low adhesion polymers can be fully or partially fluorinated or non-fluorinated. Examples of low adhesion fluorinated polymers that can be used include poly(tetrafluoro ethylene) (PTFE), poly(vinylidene fluoride)(PVDF), and poly(vinylidene fluoride-co-hexafluoropropene) (PVDF-HFP). Various brands of PTFE can be used, including any product of TEFLON family available from E. 1. DuPont de Nemours of Wilmington, Delaware. Various brands of PVDF-HFP known as SOLEF family of products, available from Solvay Fluoropolymers, Inc. of Houston, Texas, can be used, for example, SOLEF 21508 having about 85 mass % of vinylidene fluoride-derived units and about 15 mass % of hexafluoro propene-derived units. PVDF-HFP is also available from Atofina Chemicals of Philadelphia, Pennsylvania, under the trade name KYNAR. Examples of low adhesion non-fluorinated polymers that can be used include poly(n-butyl methacrylate)(PBMA), poly(methyl methacrylate)(PMMA), poly(ethyl methacrylate)(PEMA), polycarbonate, polystyrene and poly(butyleneterephthalate-co-ethylene glycol) (PBT-PEG). In some embodiments a family of PBT-PEG known as POLYACTIVE can be used. POLYACTIVE is a trade name of a PBT-PEG group of products and is available from IsoTis Corp. of Holland. In various brands of POLYACTIVE, the ratio between the units derived from ethylene glycol and the units derived from butylene terephthalate can be between about 0.67:1 and about 9:1. The molecular weight of the units derived from ethylene glycol can be between about 300 and about 4,000 Daltons. Alternatively, in some embodiments, the balloon can be modified with a high adhesion polymer to allow a bare stent or a polymer having slippery characteristics to remain on the balloon during delivery and expansion of the stent.

A variety of solvents, such as isopropyl alcohol, can be used for making the solution to be applied to the balloon, taking into account both the solubility of a drug, polymer and/or blocking agent and the ability of the solvent to wet the pores and penetrate into the balloon material. For balloons made of poly(tetrafluoroethylene), for example, preferable solvents include acetonitrile, acetone or isopropanol.

According to embodiments of the present invention, a drug or cocktail combination of drugs can be delivered to a patient using a dual mode delivery, i.e., both via a balloon and a stent. The dual mode of the delivery is believed to be particularly beneficial for the treatment of multimodal pathologies such as restenosis. One beneficial effect that can be provided by the dual mode delivery is believed to be an ability to achieve better inhibition of restenosis. The term "inhibition" refers to reduction, elimination, prevention, or treatment of restenosis, and includes delaying the onset of the cellular activity leading to the condition. The first mode of delivery provides for delivery of a drug via the balloon of the delivery catheter, and the second mode of delivery provides for the local drug delivery via a coated stent after the stent has been positioned in place and deployed. The dual mode delivery may produce a synergistic beneficial therapeutic effect compared with the effect produced by drug delivery using either mode of delivery alone. The balloon can also provide for a quick burst of a drug followed by a prolonged local administration of the same drug or another drug by a stent. When the balloon expands, the pores can open up, releasing the embedded drug. In some embodiments, the balloon can deliver a drug immediately before the time of deployment or implantation of the stent; substantially contemporaneously with the deployment or implantation of the stent; and/or immediately after the time of deployment or implantation of the stent.

Embodiments of the present invention are illustrated by the following Examples.

### Example 1 -- Simulated Experiment

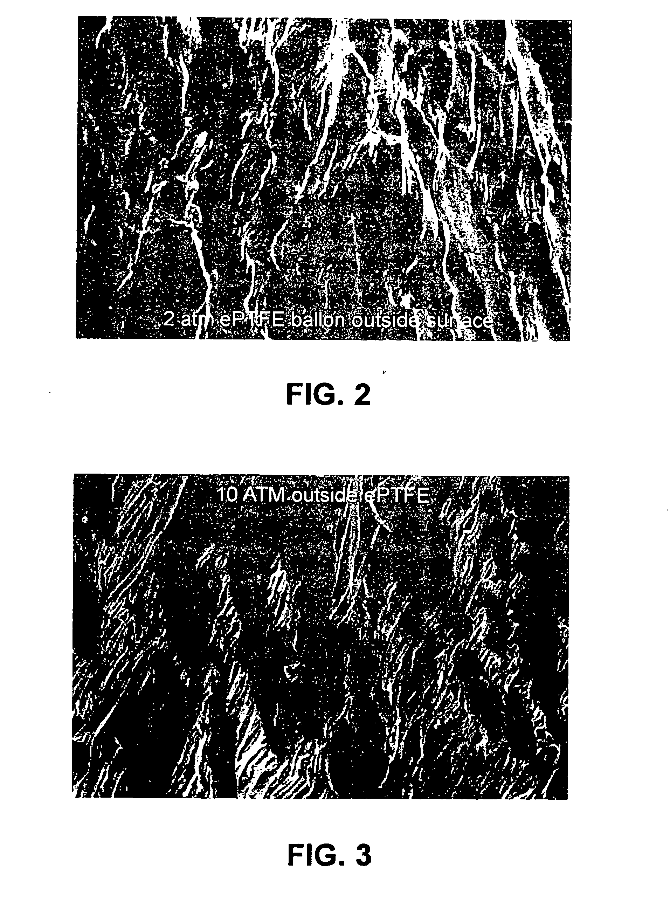

A solution containing about 2 mass % EVEROLIMUS, and the balance, acetonitrile, was prepared. One drop of a blue azo dye was added for contrast. Two balloon sub-assemblies, each containing an ePTFE balloon were made. Both sub-assemblies were immersed in the EVEROLIMUS solution for about 30 seconds and then removed and visually inspected. Very minimal blue staining was observed in each case, indicating that not more than a very small, negligible, amount of EVEROLIMUS was impregnated into the balloon membranes. The very insignificant penetration of EVEROLIMUS into the balloon can be explained by the fact that the ePTFE balloon in the uninflated state contained very few pores, as shown by FIG. 2.

The balloon of the first sub-assembly was then inflated using saline solution to about 8 atmospheres and immersed into the EVEROLIMUS solution for about 30 seconds again. Some staining of the pores took place indicating the penetration of EVEROLIMUS into the balloon wall membrane; however, the balloon burst at this pressure.

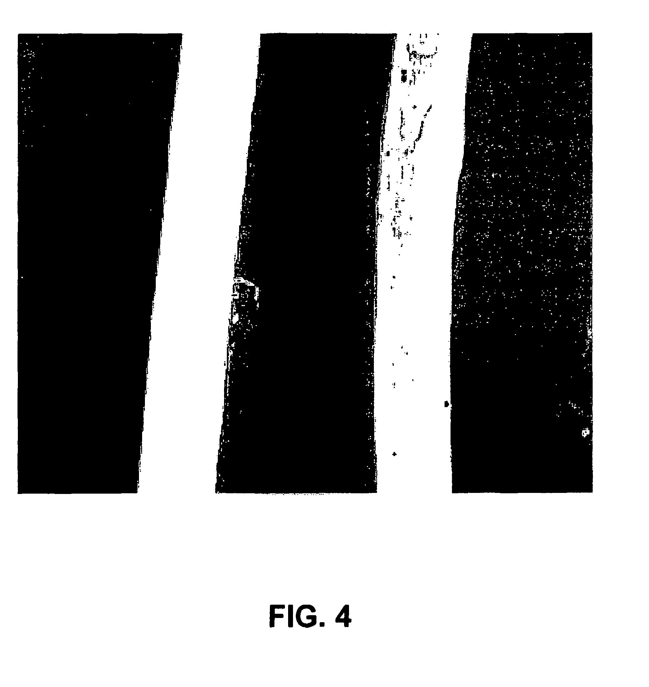

The balloon of the second sub-assembly was inflated using saline solution to about 6 atmospheres and immersed into the EVEROLIMUS solution for about 30 seconds again, followed by the visual inspection. The inspection revealed that the pores of the balloon were significantly stained by the blue dye, indicating substantial penetration of EVEROLIMUS into the wall of the balloon. Substantial penetration of EVEROLIMUS into the inflated balloon can be explained by the fact that the ePTFE balloon, in the inflated state, contained many pores, as shown by FIG. 3. The blue dye staining is also clearly shown by FIG. 4 which is an optical microphotograph where an ePTFE balloon (left) is compared with a dyed ePTFE balloon (right).

The simulated experiment, therefore, has demonstrated that EVEROLIMUS can be loaded into the pores of the balloon when an inflated balloon is immersed into an EVEROLIMUS solution. The loading of EVEROLIMUS was not achieved when the balloon was uninflated.

### Examples 2

To incorporate the drug into the balloon (e. g., made from ePTFE), the drug can be dissolved in a solvent (e. g., isoporpyl alcohol, acetone, acetonitrile) to make a drug solution having concentration between about 0.1 mass % and about 30 mass %, such as between about 2 mass % and about 10 mass %, for example, about 5 mass %. The balloon can be then inflated using a fluid, such as saline solution, followed by immersing the inflated balloon in the drug solution for about 30 seconds. The inflation pressure can be between about 6 atmospheres and about 18 atmospheres, more narrowly, between about 12 atmospheres and about 18 atmospheres, for example, about 18 atmospheres. (1 atmosphere = 10 exp5 Pascal)

While particular embodiments of the present invention have been shown and described, it will be obvious to those skilled in the art that changes and modifications can be made without departing from this invention as defined by the appended claims.

## Claims

1. A method of modifying a balloon of a catheter assembly, comprising:
creating cavities or surface depots within a wall membrane of the balloon by a manufacturing process without puncturing the balloon wall membrane, the wall membrane being made of a non-porous material;
inflating the balloon from a collapsed configuration to an inflated state; and
applying a substance to the balloon, wherein the substance is deposited within the wall membrane of the balloon.

2. The method of Claim 1, wherein the inflated state is greater than a range of an intended expanded configuration of the balloon.

3. The method of Claim 1, wherein the inflated state is less than a range of an intended expanded configuration of the balloon.

4. The method of Claim 1, wherein the balloon has an intended expanded configuration and the inflated state is a state in which the balloon has a diameter above the intended expanded configuration of the balloon but less than a diameter which will cause the balloon to be damaged or be no longer suitable for its intended use.

5. The method of Claim 1, wherein the inflated state is maintained at the same or generally the same level during the application of the substance to the balloon.

6. The method of Claim 1, wherein the inflated state is increased or decreased during the application of the substance to the balloon.

7. The method of Claim 1, additionally including pulsating the balloon to a greater and/or smaller size during the application of the substance.

8. The method of Claim 1, wherein the substance is in a fluid form or carried by a fluid carrier.

9. The method of Claim 8, wherein the method additionally comprises blowing gas at the balloon.

10. The method of Claim 8 when the substance is carried by a fluid carrier, additionally comprising removing the fluid carrier from the balloon such that a dry form of the substance is left on and/or within the wall membrane of the balloon.

11. The method of Claim 10, wherein the balloon is reduced to a deflated state or to the collapsed configuration prior to or during the process of removal of the fluid carrier.

12. The method of Claim 10, wherein the inflated state is maintained at the same or a generally same level during the removal of the fluid carrier.

13. The method of Claim 10, wherein the balloon is pulsed to a greater and/or smaller size during the removal of the fluid carrier.

14. The method of Claim 1, wherein the substance is one of or a combination of a therapeutic substance, a polymeric material and a blocking agent.

15. The method of Claim 9, wherein gas is blown contemporaneously with the application of a substance.

16. The method of Claim 9, wherein gas is blown subsequent to the application of the substance.

17. The method of Claim 1, wherein the balloon is inflated further subsequent to the application of the substance.

18. The method of Claim 8 when the substance is carried by a fluid carrier, further comprising applying an inert gas to the balloon contemporaneously or subsequently to applying the substance in the fluid carrier to the balloon.

19. The method of Claim 8 when the substance is carried by a fluid carrier, wherein the substance is dissolved in the fluid carrier.

20. The method of Claim 8 when the substance is carried by a fluid carrier, wherein the substance is saturated in the fluid carrier.

21. The method of Claim 8 when the substance is carried by a fluid carrier, wherein the substance is supersaturated in the fluid carrier.

22. The method of Claim 1, wherein the substance includes a drug.

23. The method of Claim 1, wherein the cavities or surface depots are created by laser drilling, etching, mechanical drilling or chemical drilling.

## Patentansprüche

1. Verfahren zur Modifizierung eines Ballons einer Katheteranordnung, wobei das Verfahren die folgenden Schritte aufweist:
Schaffen von Hohlräumen oder Oberflächenablagen in einer Wandmembran des Ballons mit Hilfe eines Herstellungsprozesses, ohne die Ballon-Wandmembran zu durchstechen, wobei die Wandmembran aus einem nicht-porösen Material hergestellt ist;
Aufblasen bzw. Füllen des Ballons von einer zusammengeklappten Konfiguration in einen aufgeblasenen Zustand; und
Aufbringen bzw. Applizieren einer Substanz auf den Ballon, wobei die Substanz in der Wandmembran des Ballons abgelagert wird.

2. Verfahren nach Anspruch 1, **dadurch gekennzeichnet, dass** der aufgeblasene Zustand größer als eine Abgrenzung einer beabsichtigten ausgedehnten Konfiguration des Ballons ist.

3. Verfahren nach Anspruch 1, **dadurch gekennzeichnet, dass** der aufgeblasene Zustand kleiner als eine Abgrenzung einer beabsichtigten ausgedehnten Konfiguration des Ballons ist.

4. Verfahren nach Anspruch 1, **dadurch gekennzeichnet, dass** der Ballon eine beabsichtigte ausgedehnte Konfiguration aufweist und dass der aufgeblasene Zustand ein Zustand ist, bei welchem der Ballon einen Durchmesser größer als die beabsichtigte ausgedehnte Konfiguration des Ballons aufweist, jedoch kleiner als ein Durchmesser, welcher eine Beschädigung des Ballons verursacht oder nicht länger für seinen beabsichtigten Einsatz geeignet ist.

5. Verfahren nach Anspruch 1, **dadurch gekennzeichnet, dass** der aufgeblasene Zustand auf demselben oder im Allgemeinen auf demselben Pegel während der Aufbringung bzw. Applikation der Substanz auf den Ballon gehalten wird.

6. Verfahren nach Anspruch 1, **dadurch gekennzeichnet, dass** der aufgeblasene Zustand während der Aufbringung bzw. Applikation der Substanz auf den Ballon erhöht oder verringert wird.

7. Verfahren nach Anspruch 1, welches des Weiteren die Pulsation des Ballons auf eine größere und/oder kleinere Abmessung während der Aufbringung bzw. Applikation der Substanz einschließt.

8. Verfahren nach Anspruch 1, **dadurch gekennzeichnet, dass** die Substanz in Fluidform vorliegt oder dass sie von einem Fluidträger getragen wird.

9. Verfahren nach Anspruch 8, **dadurch gekennzeichnet, dass** das Verfahren zusätzlich das Anblasen des Ballons mit Gas aufweist.

10. Verfahren nach Anspruch 8, welches in dem Fall, dass die Substanz von einem Fluidträger getragen wird, zusätzlich das Entfernen des Fluidträgers von dem Ballon aufweist, so dass eine trockene Form der Substanz auf und/oder in der Wandmembran des Ballons zurück- bzw. übrigbleibt.

11. Verfahren nach Anspruch 10, **dadurch gekennzeichnet, dass** der Ballon aufeinen entleerten Zustand oder auf die zusammengeklappte Konfiguration verringert bzw. verkleinert wird, bevor oder während der Vorgang der Beseitigung des Fluidträgers stattfindet.

12. Verfahren nach Anspruch 10, **dadurch gekennzeichnet, dass** der aufgeblasene Zustand auf demselben oder im Allgemeinen auf demselben Pegel während der Beseitigung des Fluidträgers gehalten wird.

13. Verfahren nach Anspruch 10, **dadurch gekennzeichnet, dass** der Ballon auf eine größere und/oder kleinere Abmessung während der Beseitigung des Fluidträgers gepulst wird.

14. Verfahren nach Anspruch 1, **dadurch gekennzeichnet, dass** die Substanz entweder eine therapeutische Substanz, ein Polymerwerkstoff oder ein blockierendes Mittel oder eine Kombination daraus ist.

15. Verfahren nach Anspruch 9, **dadurch gekennzeichnet, dass** zeitgleich mit der Aufbringung bzw. Applikation einer Substanz ein Gas eingeblasen wird.

16. Verfahren nach Anspruch 9, **dadurch gekennzeichnet, dass** Gas im Anschluss an die Aufbringung bzw. Applikation der Substanz eingeblasen wird.

17. Verfahren nach Anspruch 1, **dadurch gekennzeichnet, dass** der Ballon im Anschluss an die Aufbringung bzw. Applikation der Substanz weiter aufgeblasen wird.

18. Verfahren nach Anspruch 8, **dadurch gekennzeichnet, dass** wenn die Substanz von einem Fluidträger getragen wird, das Verfahren des Weiteren die Anwendung von Edelgas an dem Ballon zeitgleich mit oder im Anschluss an die Aufbringung bzw. Applikation der Substanz in dem Fluidträger auf den Ballon aufweist.

19. Verfahren nach Anspruch 8, **dadurch gekennzeichnet, dass** wenn die Substanz von einem Fluidträger getragen wird, die Substanz in dem Fluidträger aufgelöst wird.

20. Verfahren nach Anspruch 8, **dadurch gekennzeichnet, dass** wenn die Substanz von einem Fluidträger getragen wird, die Substanz in dem Fluidträger gesättigt ist.

21. Verfahren nach Anspruch 8, **dadurch gekennzeichnet, dass** wenn die Substanz von einem Fluidträger getragen wird, die Substanz in dem Fluidträger übersättigt ist.

22. Verfahren nach Anspruch 1, **dadurch gekennzeichnet, dass** die Substanz ein Medikament einschließt.

23. Verfahren nach Anspruch 1, **dadurch gekennzeichnet, dass** die Hohlräume oder Oberflächenablagen durch Laserbohren, Ätzen, mechanisches Bohren oder chemisches Bohren erzeugt werden.

## Revendications

1. Procédé de modification d'un ballonnet d'un ensemble cathéter, comprenant:
créer des cavités ou des dépôts de surface dans une membrane de paroi du ballonnet par un processus de fabrication sans ponctionner la membrane de paroi du ballonnet, la membrane de paroi étant réalisée en un matériau non poreux;
gonfler le ballonnet d'une configuration affaissée en un état gonflé; et
appliquer une substance au ballonnet, où la substance est déposée dans la membrane de paroi du ballonnet.

2. Procédé selon la revendication 1, dans lequel l'état gonflé est plus grand qu'une plage de configuration expansée prévue du ballonnet.

3. Procédé selon la revendication 1, dans lequel l'état gonflé est plus petit qu'une plage de configuration expansée prévue du ballonnet.

4. Procédé selon la revendication 1, dans lequel le ballonnet a une configuration expansée prévue, et l'état gonflé est un état dans lequel le ballonnet a un diamètre au-dessus de la configuration expansée prévue du ballonnet mais inférieur à un diamètre qui amènera le ballonnet à être endommagé ou à ne pas plus convenir à son utilisation prévue.

5. Procédé selon la revendication 1, dans lequel l'état gonflé est maintenu au même niveau ou généralement au même niveau durant l'application de la substance au ballonnet.

6. Procédé selon la revendication 1, dans lequel l'état gonflé est augmenté ou diminué durant l'application de la substance au ballonnet.

7. Procédé selon la revendication 1, incluant additionnellement la pulsation du ballonnet à une plus grande et/ou plus petite taille durant l'application de la substance.

8. Procédé selon la revendication 1, dans lequel la substance se présente sous forme de fluide ou est portée par un support de fluide.

9. Procédé selon la revendication 8, dans lequel le procédé comprend additionnellement le soufflage de gaz au ballonnet.

10. Procédé selon la revendication 8, lorsque la substance est portée par un support de fluide, comprenant additionnellement le retrait du support de fluide du ballonnet de sorte qu'une forme sèche de la substance subsiste sur et/ou dans la membrane de paroi du ballonnet.

11. Procédé selon la revendication 10, dans lequel le ballonnet est réduit à un état dégonflé ou à la configuration affaissée avant ou durant le processus de retrait du support de fluide.

12. Procédé selon la revendication 10, dans lequel l'état gonflé est maintenu au même ou généralement au même niveau durant le retrait du support de fluide.

13. Procédé selon la revendication 10, dans lequel le ballonnet est pulsé à une taille plus grande et/ou plus petite durant le retrait du support de fluide.

14. Procédé selon la revendication 1, dans lequel la substance est un de ou une combinaison d'une substance thérapeutique, d'un matériau polymère et d'un agent de blocage.

15. Procédé selon la revendication 9, dans lequel le gaz est soufflé simultanément avec l'application d'une substance.

16. Procédé selon la revendication 9, dans lequel le gaz est soufflé à la suite de l'application de la substance.

17. Procédé selon la revendication 1, dans lequel le ballonnet est gonflé en outre à la suite de l'application de la substance.

18. Procédé selon la revendication 8, lorsque la substance est portée par un support de fluide, comprenant en outre l'application d'un gaz inerte au ballonnet simultanément ou à la suite de l'application de la substance dans le support de fluide au ballonnet.

19. Procédé selon la revendication 8, lorsque la substance est portée par un support de fluide, dans lequel la substance est dissoute dans le support de fluide.

20. Procédé selon la revendication 8, lorsque la substance est portée par un support de fluide, dans lequel la substance est saturée dans le support de fluide.

21. Procédé selon la revendication 8, lorsque la substance est portée par un support de fluide, dans lequel la substance est supersaturée dans le support de fluide.

22. Procédé selon la revendication 1, dans lequel la substance comporte un médicament.

23. Procédé selon la revendication 1, dans lequel les cavités ou dépôts de surface sont créés par forage au laser, attaque, forage mécanique ou forage chimique.
